(19) Europäisches Patentamt
European Patent Office
Office européen des brevets

(11) Numéro de publication: **0 071 521**
**B1**

(12) # FASCICULE DE BREVET EUROPEEN

(45) Date de publication du fascicule du brevet: **04.12.85**

(51) Int. Cl.[4]: **C 07 D 401/04**, A 61 K 31/44

(21) Numéro de dépôt: **82401358.5**

(22) Date de dépôt: **21.07.82**

(54) **Nouveaux dérivés du 2-oxo-pyrid-3-yl ou pipéridin-3-yl indole, leurs sels, procédé de préparation, application à titre de médicaments et compositions les renfermant.**

(30) Priorité: **24.07.81 FR 8114429**

(43) Date de publication de la demande:
**09.02.83 Bulletin 83/6**

(45) Mention de la délivrance du brevet:
**04.12.85 Bulletin 85/49**

(84) Etats contractants désignés:
**AT BE CH DE FR GB IT LI LU NL SE**

(56) Documents cités:
**EP - A - 0 021 924**

**THE CHEMISTRY OF HETEROCYCLIC COMPOUNDS, Indoles Tome II, pages 142,143, Wiley-Interscience, New York, USA**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Nedelec, Lucien, 45, boulevard de L'Ouest, F-93340 Le Raincy (FR)**
Inventeur: **Guillaume, Jacques, 15, avenue du Belvédère Appt.1904, F-93310 Le Pré-Saint-Gervais (FR)**
Inventeur: **Dumont, Claude, 33, rue du Maréchal Vaillant, F-94130 Nogent-sur-Marne (FR)**

(74) Mandataire: **Petranker, Léon et al, boîte postale no 9 102, route de Noisy, F-93230 Romainville (FR)**

EP 0 071 521 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux dérivés du 2-oxo pyrid-3-yl ou pipéridin-3-yl indole ainsi que leurs sels, le procédé de préparation, l'application à titre de médicaments de ces nouveaux dérivés et les compositions pharmaceutiques les renfermant.

Des dérivés du pipéridin-et du tétrahydropyridin-3-yl indole doués notamment de propriétés stimulantes dopaminergiques ont déjà été décrits dans la demande de brevet européen n° 0 021 924. La présente demande concerne des dérivés du 2-oxo indole doués également de propriétés stimulantes dopaminergiques, très marquées au niveau de la prolactine.

L'invention a pour objet les dérivés du 2-oxo indole ainsi que leurs sels d'addition avec les acides minéraux ou organiques, caractérisés en ce qu'ils répondent à la formule générale (I):

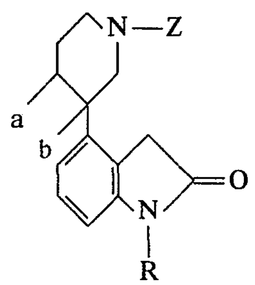

(I)

dans laquelle

- R représente un atome d'hydrogène, un radical alkyle renfermant de 1 à 8 atomes de carbone, ou un radical aralkyle renfermant de 7 à 12 atomes de carbone,
- Z représente un atome d'hydrogène, un radical méthyle, un radical alkyle ou hydroxyalkyle renfermant de 2 à 8 atomes de carbone, un radical aryloxyalkyle renfermant de 7 à 12 atomes de carbone, un radical aralkyle renfermant de 7 à 12 atomes de carbone éventuellement substitué par un ou plusieurs atomes d'halogène, par un ou plusieurs radicaux alkoxy renfermant de 1 à 4 atomes de carbone, par un plusieurs radicaux alkyles renfermant de 1 à 4 atomes de carbone, ou par un ou plusieurs radicaux OH, $CF_3$, $OCF_3$, $NO_2$ ou $NH_2$, ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone et,
- ou bien a et b représentent chacun un atome d'hydrogène,
- ou bien a et b forment sensemble une liaison carbone-carbone, étant entendu que lorsque Z représente un radical alkényle ou alkynyle, la liaison multiple ne peut pas se trouver entre les carbones en $\alpha$ et en $\beta$ de l'atome d'azote.

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide de méthane sulfonique et arylsulfoniques, tels que l'acide benzènesulfonique.

Lorsque R représente un radical alkyle, il s'agit de préférence du radical méthyle, éthyle, propyle, isopropyle, butyle, isobutyle ou n-pentyle.

Lorsque R représente un radical aralkyle, il s'agit de préférence du radical benzyle.

Lorsque Z représente un radical alkyle ou hydroxyalkyle, »alkyle« représente de préférence un radical éthyle, n-propyle ou isopropyle, n-butyle ou isobutyle, n-pentyle ou n-hexyle.

Lorsque Z représente un radical aryloxyalkyle, il s'agit de préférence du radical phényloxyéthyle ou phényloxypropyle.

Lorsque Z représente un radical aralkyle, il s'agit de préférence d'un radical benzyle ou phénéthyle.

Lorsque Z représente un radical aralkyle substitué par un ou plusieurs atomes d'halogène, on entend de préférence par halogène un atome de chlore ou de brome.

Lorsque Z représente un radical aralkyle substitué par un ou plusieurs radicaux alcoxy, on entend de préférence par alcoxy un radical méthoxy ou éthoxy.

Lorsque Z représente un radical aralkyle substitué par un ou plusieurs radicaux alkyles, on entend de préférence par alkyle un radical méthyle ou éthyle.

Lorsque Z représente un radical cycloalkylalkyle, il s'agit de préférence d'un radical cyclopropyle alkyle, par exemple du radical cyclopropylméthyle, cyclopropyléthyle, ou cyclopropyl-n-propyle.

Lorsque Z représente un radical alkényle, il s'agit de préférence du radical allyle.

Lorsque Z représente un radical alkynyle, il s'agit de préférence du radical propargyle.

Parmi les produits objet de l'invention, on peut citer notamment, les dérivés répondant à la formule (I) ci-dessus, ainsi que leurs sels d'addition avec les acides minéraux ou organiques, caractérisés en ce que, dans ladite formule (I), R représente un atome d'hydrogène ou un radical alkyle renfermant de 1 à

8 atomes de carbone.

Parmi ceux-ci, on peut citer plus particulièrement les dérivés caractérisés en ce que, dans ladite formule (I), Z représente un atome d'hydrogène ou un radical alkyle renfermant de 1 à 8 atomes de carbone, ainsi que leurs sels d'addition avec les acides minéraux ou organiques, et tout particulièrement

– la 1,3-dihydro 4-(1-propyl 1,2,5,6-tétrahydropyridin-3-yl) 2H indol-2-one,
– la 1,3-dihydro 4-(pipéridin-3-yl) 2H indol-2-one,

ainsi que leurs sels d'addition avec les acides minéraux ou organiques.

L'invention a également pour objet un procédé de préparation des dérivés, tels que définis par la formule (I) ci-dessus, ainsi que de leurs sels, caractérisé en ce que l'on hydrolyse un produit de formule (II):

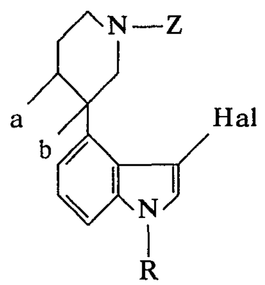

(II)

dans laquelle Hal représente un atome de chlore ou de brome, et R, Z, a et b ont la signification déjà indiquée, pour obtenir un produit de formule générale (I):

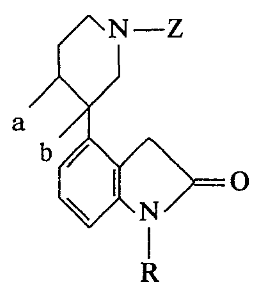

(I)

dans laquelle R, Z, a et b ont la signification déjà indiquée, que l'on isole et salifie si désiré.

L'hydrolyse du produit de formule II est réalisée de préférence, à l'aide d'un acide minéral en solution aqueuse, tel que l'acide phosphorique, l'acide sulfurique ou de préférence l'acide chlorhydrique. Cette solution peut être utilisée concentrée, mais de préférence diluée, par exemple décinormale.

L'invention a également pour objet une variante du procédé de préparation des dérivés de formule (I), variante caractérisée en ce que l'on hydrolyse un produit de formule (III):

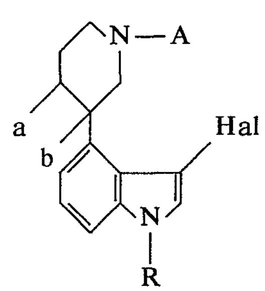

(III)

dans laquelle Hal, R, a et b ont la signification déjà indiquée et A représente un atome d'hydrogène, un groupement protecteur éliminable par hydrolyse acide ou un groupement benzyle, pour obtenir un produit de formule ($I_A$):

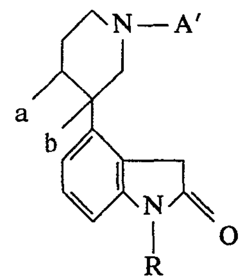

($I_A$)

dans laquelle R, a et b ont la signification déjà indiquée et A' représente un atome d'hydrogène ou un groupement benzyle, correspondant à un produit de formule (I) dans laquelle Z représente un atome d'hydrogène ou un groupement benzyle, composé de formule ($I_A$) que l'on soumet, le cäs échéant, à un agent de clivage du groupement benzyle, pour obtenir un produit de formule ($I_B$):

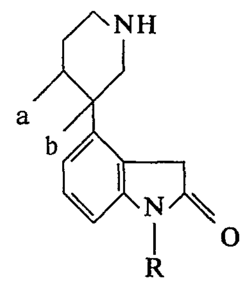

($I_B$)

dans laquelle R, a et b ont la signification déjà indiquée, que, le cas échéant, l'on soumet à l'action d'un agent capable d'introduire un radical Z', Z' ayant la même signification que Z, à l'exception d'hydrogène, pour obtenir un produit de formule ($I_C$):

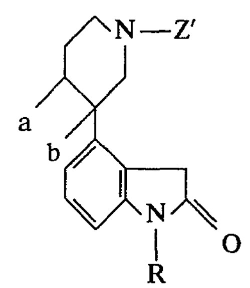

($I_C$)

dans laquelle R, Z', a et b ont la signification déjà indiquée, composé de formule ($I_A$), ($I_B$) ou ($I_C$) que, le cas échéant, l'on salifie.

L'hydrolyse du produit de formule (III) est effectuée de préférence dans les mêmes conditions que celles de l'hydrolyse du produit de formule (II).

Si a et b ne représentent pas ensemble und liaison carbone-carbone, l'agent de clivage du groupement benzyle porté par le noyau pipéridinyle est de préférence l'hydrogène en présence d'un catalyseur, par exemple, l'hydrogène en présence de palladium,

- pour cliver le groupement benzyle dans le cas où a et b représentent ensemble une liaison carbone-carbone, on fait préférence réagir le chloroformiate d'éthyle pour former le carbamate d'éthyle correspondant que l'on hydrolyse de préférence en milieu alcalin,
- l'introduction du radical Z' est réalisée de préférence au moyen d'un halogénure Z'-Hal' dans lequel Hal' représente un atome de chlore, de brome ou d'iode.

Les dérivés de formule (I) présentent un caractère basique. On peut avantageusement préparer les sels d'addition des dérivés de formule (I), en faisant réagir, en proportions sensiblement stoechiométriques, un acide minéral ou organique avec lesdits dérivés de formule (I). Les sels peuvent être préparés sans isoler les bases correspondantes.

Les dérivés objet de la présente invention, possèdent de très intéressantes propriétés pharmacologiques; ils sont doués notamment de remarquables propriétés stimulantes dopaminergiques et antianoxiques. Ces propriétés sont illustrées plus loin dans la partie expérimentale.

Ces propriétés justifient l'utilisation des dérivés du 2-oxo indole de formule (I), ainsi que de leurs sels pharmaceutiquement acceptables, à titre de médicaments.

La présente demande a ainsi également pour objet, l'application, à titre de médicaments, des dérivés du 2-oxo indole, tels que définis par la formule (I), ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du 2-oxo indole répondant à la formule (I), dans laquelle R représente un atome d'hydrogène ou un radical alkyle renfermant de 1 à 8 atomes de carbone, ainsi que par leurs sels d'addition avec les acides pharmaceutiquement acceptables.

Parmi ceux-ci, on retient notamment ceux répondant à la formule (I), dans laquelle Z représente un atome d'hydrogène ou un radical alkyle renfermant de 1 à 8 atomes de carbone, ainsi que leurs sels d'addition avec les acides pharmaceutiquement acceptables.

Parmi ces derniers, on retient tout particulièrement la 1,3-dihydro 4-(1-propyl 1,2,5,6-tétrahydro-pyridin-3-yl) 2H indol 2-one, la 1,3-dihydro 4-(pipéridin-3-yl) 2H indol-2-one, ainsi que leurs sels d'addition avec les acides pharmaceutiquement acceptables.

On peut encore citer la 1,3-dihydro 4-(1-méthyl pipéridin-3-yl) 2H indol-2-one et la 1,3-dihydro 4-(1-propyl pipéridin-3-yl) 2H indol-2-one, ainsi que leurs sels d'addition avec les acides pharmaceutiquement acceptables.

Les médicaments selon l'invention trouvent leur emploi par exemple dans le traitement de très nombreuses maladies ou désordres pathologiques divers; ils peuvent, par exemple, être utilisés dans le traitement de l'hypersécrétion de prolactine par l'antéhypophyse, et de ce fait, dans le traitement de l'hypogonadisme de la femme et de l'homme; ils peuvent également être utilisés dans le traitement des syndromes neurologiques d'origine extrapyramidale, par exemple dans le traitement de la maladie de Parkinson et dans le traitement des syndromes parkinsonniens post-encéphalitiques.

Ils peuvent être également utilisés dans le traitement de la sénescence cérébrale et en gériatrie.

La dose usuelle, variable selon le dérivé utilisé, le sujet traité et l'affection en cause, peut être, par exemple, de 5 mg à 100 mg par jour, par voie orale chez l'homme du dérivé de l'exemple 1 pour le traitement de la maladie de Parkinson.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiquement couramment utilisées en médecine humaine, comme par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de départ de formule (II) sont connus ou peuvent être préparés comme indiqué dans le brevet français n° 2 458 549 ou la demande de brevet européen n° 0 021 924.

Les exemples qui suivent illustrent l'invention.

## Exemple 1

### Chlorhydrate de 1,3-dihydro 4-(1-propyl 1,2,5,6-tétrahydropyridin-3-yl)2H-indole-2-one

On agite 5 g de 3-bromo 4-(1-propyl 1,2,5,6-tétrahydropyridin-3-yl)1H indole dans 100 $cm^3$ de solution aqueuse d'acide chlorhydrique N pendant 3 heures, dilue à l'eau, alcalinise à l'aide d'une solution aqueuse de soude jusqu'à pH basique, relargue au carbonate de potassium, extrait à l'acétate d'éthyle, évapore à sec et obtient 4,1 g de base.

### Formation de chlorhydrate

On dissout la base ci-dessus dans 100 $cm^3$ d'acétate d'éthyle, ajoute une solution d'acétate d'éthyle chlorhydrique jusqu'à pH acide, filtre, lave à l'acétate d'éthyle, sèche sous pression réduite et obtient 4,5 g du produit. F = 264°C.

Analyse (après recristallisation dans un mélange isopropanol-méthanol 2-1) $C_{16}H_{20}N_2O$ = 292,810

| | Calculé | Trouvé |
|---|---|---|
| C % = | 65,63 | 65,4 |
| H % = | 7,23 | 7,3 |
| N % = | 9,57 | 9,3 |
| Cl % = | 12,11 | 11,9 |

Le 3-bromo 4-(1-propyl 1,2,5,6-tétrahydropyridin-3-yl)1H-indole de départ peut être préparé comme suit:

On agite pendant 3 heures sous atmosphère inerte 9 g de 4-(1-propyl 1,2,5,6-tétrahydropyridin-4-yl)1H-indole dans 150 $cm^3$ de dioxane, avec 7 g de N-bromosuccinimide, reprend à l'eau, extrait à l'acétate d'éthyle, lave à l'eau puis avec une solution aqueuse saturée de chlorure de sodium, sèche, élimine les solvants à 40°C sous pression réduite, purifie par chromatographie sur silice (Eluant: cyclohexane-chloroforme-triéthylamine 6-3-1), isole les fractions de Rf = 0,2, élimine les solvants — sous pression réduite, reprend à l'éther isopropylique, filtre, sèche sous pression réduite, et obtient 5,5 g du produit attendu. F = 156°C.

Analyse: $C_{16}H_{19}BrN_2$ = 319,251

| | Calculé | Trouvé |
|---|---|---|
| C % = | 60,20 | 60,4 |
| H % = | 6,00 | 6,0 |
| Br % = | 25,03 | 25,0 |
| N % = | 8,77 | 8,7 |

## Exemple 2

### Chlorhydrate de 1,3-dihydro 4-(pipéridin-3-yl) 2H indol-2-one

On chauffe au reflux, pendant 3 heures, un mélange de 11 g de 3-chloro 4-(1-trifluoroacétyl pipéridin-3-yl) 1H-indole et 600 $cm^3$ d'acide chlorhydrique N. On refroidit ensuite à 20—25°C, alcalinise par addition de soude concentrée, sature par du carbonate de potassium et extrait à l'acétate d'éthyle. On obtient après décantation de la phase organique, séchage, et élimination du solvant, 3,65 g de produit brut que l'on chromatographie sur silice en éluant au mélange chloroforme-méthanol-triéthylamine (7-2-1). On obtient 3,6 g de produit.

### Formation du chlorhydrate

On dissout le produit ci-dessus dans 50 $cm^3$ d'acétate d'éthyle et 50 $cm^3$ d'isopropanol, puis ajoute une solution saturée d'acide chlorhydrique dans l'acétate d'éthyle. On dissout le sel formé — par addition de 300 $cm^3$ d'isopropanol et 100 $cm^3$ de méthanol et chauffage au reflux. On filtre à chaud, concentre à 100 $cm^3$, refroidit, essore et sèche les cristaux. On obtient 3,6 g de produit attendu. F > 260°C.

Analyse: $C_{13}H_{17}ClN_2O$ = 252,745

| | | | | |
|---|---|---|---|---|
| Calculé: | C% 61,78 | H% 6,78 | N% 11,08 | Cl% 14,03 |
| Trouvé: | C% 61,5 | H% 6,9 | N% 10,9 | Cl% 14,0 |

Le produit de départ de l'exemple 2 a été préparé comme suit.

### Stade A

### 4-(1-trifluoroacétyl pipéridin-3-yl) 1H indole

On mélange 7 g de 4-(pipéridin-3-yl) 1H indole, 70 $cm^3$ de chloroforme et 48,6 $cm^3$ de triéthylamine, puis introduit à 0 +10°C, 24,3 $cm^3$ d'anhydride trifluoroacétique et maintient sous agitation pendant 30 minutes. On ajoute de l'eau, décante, extrait au chloroforme, sèche les phases organiques réunies, élimine le solvant, purifie le résidu sur silice en éluant au mélange cyclohexane-chloroforme-triéthylamine (6-3-1) et obtient 9,2 g de produit attendu. F = 170°C.

### Stade B

### 3-chloro 4-(1-trifluoroacétyl-pipéridin-3-yl) 1H-indole

On maintient sous agitation pendant 20 heures à 20°C un mélange de 10 g de produit tel qu'obtenu au stade A, 300 $cm^3$ de dioxanne et 5 g de N-chlorosuccinimide. On ajoute de l'eau, extrait à l'acétate

d'éthyle, lave la phase organique à l'eau et à l'eau salée, sèche, évapore le solvant, purifie le résidu sur silice en éluant au mélange cyclohexane-chloroforme-triéthylamine (6-3-1) et obtient 11 g de produit attendu utilisé tel quel pour la suite de la synthèse.

## Exemple 3

### Chlorhydrate de 1,3-dihydro 4-(1-propyl pipéridin-3-yl) 2H-indol-2-one

On utilise au départ la base du produit obtenu à l'exemple 2 et on traite ce produit par l'iodure de propyle dans le diméthylformamide en présence de carbonate de sodium. On obtient le produit attendu sous forme de base et on en prépare le chlorhydrate dans l'éther. F = 260°C après recristallisation dans le mélange isopropanol-méthanol.

## Exemple 4

### 1,3-dihydro 4-(1-méthyl pipéridin-3-yl) 2H-indol-2-one

On opère comme à l'exemple 3 en utilisant l'iodure de méthyle. On obtient le produit attendu (base) que l'on recristallise dans le benzène. F = 160°C.

## Exemple 5

On a préparé des comprimés répondant à a formule:

| | |
|---|---|
| – chlorhydrate de 1,3 dihydro 4-(1-propyl 1,2,5,6-tétra-hydropyridin-3-yl)2H-indol-2-one: | 10 mg |
| – excipient q. s. pour un comprimé terminé à | 100 mg |

(détail de l'excipient: lactose, amidon, talc, stéarate de magnésium).

## Etude Pharmacologique

### Comportement de rotation après lésion unilatérale du faisceau nigrostriatal par la 6-hydroxydopamine

#### Technique

La lésion est effectuée chez des rats mâles de 220 g environ par injection unilatérale dans le faisceau dopaminergique nigrostrié, de 8 ug de 6-hydroxydopamine en solution à 2 μg/μl (U. Ungerstedt, Acta Physiol. Scand. 1971, 82, suppl. 367, 69—93).

Chez de tels animaux, les agonistes dopaminergiques directs, tels que l'apomorphine, administrés par voie générale telle que la voie intraveineuse ou la voie intrapéritonéale, entraînent un comportement de rotation dans la direction contralatérale au côté lésé.

Le composé étudié est administré au moins 5 semaines après la lésion. Les animaux sont placés dans un rotomètre automatisé qui permet de compter le nombre de rotations effectuées par chaque animal dans les deux sens.

Le produit de l'exemple 1 administré par voie intrapéritonéale, a provoqué de 400 à 700 rotations contralatérales par animal, dès la dose de 2 mg/kg.

### Etude de l'anoxie hypobare chez la souris

On utilise des souris mâles d'un poids de 20 à 22 g, à jeun depuis 5 heures, réparties par groupes de 10 animaux. On détermine le temps de survie des souris placées dans une enceinte hermétique dans laquelle on amène la pression à 90 mm de mercure au moyen d'une pompe. Le produit étudié est administré par voie orale, 30 minutes avant l'essai. Les témoins ne reçoivent aucun produit.

Les résultats suivants correspondent à l'augmentation du temps de survie exprimés en pourcentage par rapport aux animaux témoins:

| | | |
|---|---|---|
| Produit de l'exemple 1 | 10 mg/kg | 50% |
| | 2 mg/kg | 5% |

Inhibition de la prolactine plasmatique in vivo

On place des râts mâles de souche Sprague-Dawley par deux dans des cages et les met pendant une semaine dans une pièce insonorisée, à température contrôlée à 22 $\pm 2°C$, soumise à un nycthémère artificiel de 14 heures de jour et 10 heures de nuit. On place sous anesthésie un cathéter dans la veine cave supérieure droite des rats, puis leur administre, 48 heures après, une injection intrapéritonéale de 5 mg/kg de réserpine, suivie d'une administration orale de 5 mg/kg du produit testé.

La prolactine plasmatique est mesurée par la méthode radio immunologique décrite par Euvrard et al. (Neuropharmacology, 19, 379 (1980)), sur des prélèvements sanguins de 0,7 ml.

Les résultats sont exprimés en durée d'inhibition de la prolactine plasmatique.

Les résultats obtenus sont les suivants.

Les produit de l'example 2 inhibe la prolactine plasmatique pendant une durée d'environ 20 heures.

Inhibition de la sécrétion de prolactine sur des cellules de rat in vitro

On prépare des cultures primaires de cellules pituitaires antérieures de rat, selon la technique décrite par Drouin et Labrie (Endocrinology 98, 1528 (1976)). Après 4 heures d'incubation avec le produit testé, ou sans produit pour les témoins on mesure la prolactine présente dans le milieu par la méthode radio-immunologique décrite par Euvrard et al. (Neuropharmacology 19, 379 (1980)).

Les résultats sont exprimés en DE 50: dose inhibant 50% de la sécrétion de prolactine par comparaison aux témoins.

Les résultats obtenus sont les suivants.

La DE 50 du produit de l'exemple 2 est égale à 2 nMoles; le produit de l'exemple 2 est donc un puissant inhibiteur de la prolactine.

Etude de la toxicité aiqüe

On a évalué la dose létale $DL_0$ des composés des exemples 1 et 2 après administration par voie orale chez la souris.

On appelle $DL_0$ la dose maximale ne provoquant aucune mortalité au bout de 8 jours.

Le résultat obtenu est le suivant:

$DL_0 = 200$ mg/kg pour le produit de l'exemple 1 et supérieur à 400 mg/kg pour le produit de l'exemple 2.

**Revendications pour les Etats contractants: BE, CH, DE, FR, GB, IT, LI, LU, NL, SE**

1. Les dérivés du 2-oxo indole, ainsi que leurs sels d'addition avec les acides minéraux ou organiques, caractérisés en ce qu'ils répondent à la formule générale (I):

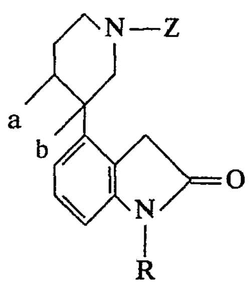

(I)

dans laquelle

- R représente un atome d'hydrogène, un radical alkyle renfermant de 1 à 8 atomes de carbone, ou un radical aralkyle renfermant de 7 à 12 atomes de carbone,
- Z représente un atome d'hydrogène, un radical méthyle, un radical alkyle ou hydroxyalkyle renfermant de 2 à 8 atomes de carbone, un radical aryloxyalkyle renfermant de 7 à 12 atomes de carbone, un radical aralkyle renfermant de 7 à 12 atomes de carbone éventuellement substitué par un ou plusieurs atomes d'halogène, par un ou plusieurs radicaux alcoxy renfermant de 1 à 4 atomes de carbone, par un ou plusieurs radicaux alkyles renfermant de 1 à 4 atomes de carbone, ou par un ou plusieurs radicaux OH, $CF_3$, $OCF_3$, $NO_2$ ou $NH_2$, ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone et,
- ou bien a et b représentent chacun un atome d'hydrogène,

– ou bien a et b forment ensemble une liaison carbone-carbone, étant entendu que lorsque Z représente un radical alkényle ou alkynyle, la liaison multiple ne peut pas se trouver entre les carbones en $\alpha$ et en $\beta$ de l'atome d'azote.

2. Les dérivés du 2-oxo indole répondant à la formule (I) telle que définie à la revendication 1, ainsi que leurs sels d'addition avec les acides minéraux ou organiques, caractérisés en ce que R représente un atome d'hydrogène ou un radical alkyle renfermant de 1 à 8 atomes de carbone.

3. Les dérivés du 2-oxo indole selon la revendication 2, ainsi que leurs sels d'addition avec les acides minéraux ou organiques, caractétisés en ce que Z représente un atome d'hydrogène ou un radical alkyle renfermant de 1 à 8 atomes de carbone.

4. L'un quelconque des dérivés de formule (I), telle que dérinie à la revendication 1, dont les noms suivent:

– la 1,3-dihydro 4-(1-propyl 1,2,5,6-tétrahydropyridin-3-yl) 2H indol-2-one,
– la 1,3-dihydro 4-(pipéridin-3-yl) 2H indol-2-one,

ainsi que leurs sels d'addition avec les acides minéraux ou organiques.

5. Procédé de préparation des dérivés du 2-oxo-indole de formule I telle que définie à la revendication 1, ainsi que de leurs sels d'addition avec les acides minéraux ou organiques, caractérisé en ce que l'on hydrolyse un produit de formule II

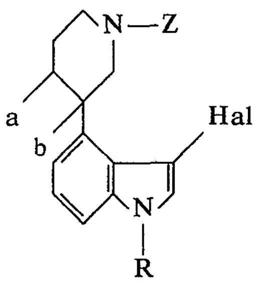

(II)

dans laquelle Hal représente un atome de chlore ou de brome, et R, Z, a et b ont la signification déjà indiquée, pour obtenir un produit de formule générale (I):

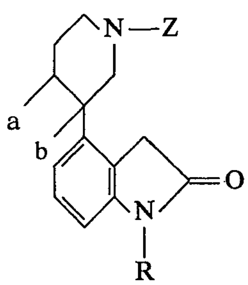

(I)

dans laquelle R, Z, a et b ont la signification déjà indiquée, que l'on isole et salifie si désiré.

6. Variante du procédé selon la revendication 5, caractérisée en ce que l'on hydrolyse un produit de formule (III):

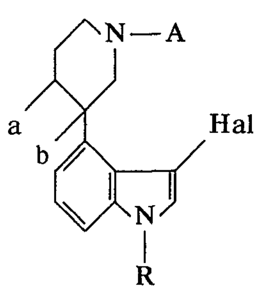

(III)

dans laquelle Hal, R, a et b ont la signification déjà indiquée à la revendication 5 et A représente un atome d'hydrogène, un groupement protecteur éliminable par hydrolyse acide ou un groupement benzyle, pour obtenir un produit de formule ($I_A$):

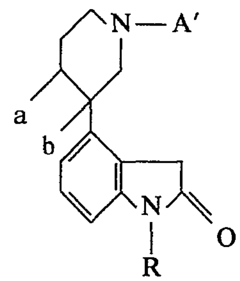

($I_A$)

dans laquelle R, a et b ont la signification déjà indiquée et A' représente un atome d'hydrogène ou un groupement benzyle, correspondant à un produit de formule (I) dans laquelle Z représente un atome d'hydrogène ou un groupement benzyle, composé de formule ($I_A$) que l'on soumet, le cas échéant, à un agent de clivage du groupement benzyle, pour obtenir un produit de formule ($I_B$):

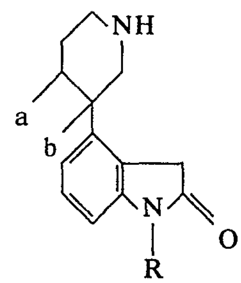

($I_B$)

dans laquelle R, a et b ont la signification déjà indiquée que, le cas échéant, l'on soumet à l'action d'un agent capable d'introduire un radical Z', Z' ayant la même signification que Z, à l'exception d'hydrogène, pour obtenir un produit de formule ($I_C$):

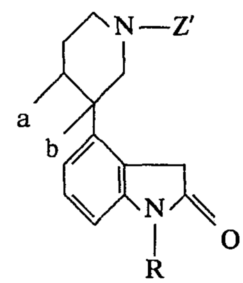

($I_C$)

dans laquelle R, Z', a et b ont la signification déjà indiquée, composé de formule ($I_A$), ($I_B$) ou ($I_C$) que, le cas échéant, l'on salifie.

7. Procédé selon la revendication 5 ou 6, caractérisé en ce que l'hydrolyse est réalisée à l'aide d'un acide minéral en solution aqueuse.

8. Médicaments, caractérisés en ce qu'ils sont constitués par les dérivés du 2-oxo indole tels que définis par la formule (I) de la revendication 1, ainsi que par leurs sels d'addition avec les acides pharmaceutiquement acceptables.

9. Médicaments, caractérisés en ce qu'ils sont constitués par les dérivés du 2-oxo indole, tels que définis dans l'une des revendications 2 ou 3, ainsi que par leurs sels d'addition avec les acides pharmaceutiquement acceptables.

10. Médicaments, caractérisés en ce qu'ils sont constitués par les dérivés du 2-oxo indole, tels que définis à la revendication 4, ainsi que par leurs sels d'addition avec les acides pharmaceutiquement acceptables.

11. Compositions pharmaceutiques, caractérisés en ce qu'elles renferment, à titre de principe actif, l'un au moins des médicaments, tels que définis à l'une des revendications 8, 9 ou 10.

**Revendications pour l'Etat contractant: AT**

1. Procédé de préparation des dérivés du 2-oxo indole, ainsi que de leurs sels d'addition avec les acides minéraux ou organiques, répondant à la formule générale (I):

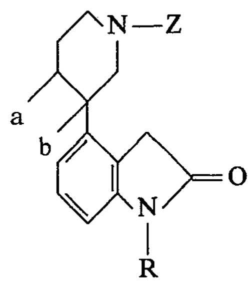

(I)

dans laquelle

- R représente un atome d'hydrogène, un radical alkyle renfermant de 1 à 8 atomes de carbone, ou un radical aralkyle renfermant de 7 à 12 atomes de carbone,
- Z représente un atome d'hydrogène, un radical méthyle, un radical alkyle ou hydroxyalkyle renfermant de 2 à 8 atomes de carbone, un radical aryloxyalkyle renfermant de 7 à 12 atomes de carbone, un radical aralkyle renfermant de 7 à 12 atomes de carbone éventuellement substitué par un ou plusieurs atomes d'halogène, par un ou plusieurs radicaux alcoxy renfermant de 1 à 4 atomes de carbone, par un ou plusieurs radicaux alkyles renfermant de 1 à 4 atomes de carbone, ou par un ou plusieurs radicaux OH, $CF_3$, $OCF_3$, $NO_2$ ou $NH_2$, ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone et,
- ou bien a et b représentent chacun un atome d'hydrogène,
- ou bien a et b forment ensemble une liaison carbone-carbone, étant entendu que lorsque Z représente un radical alkényle ou alkynyle, la liaison multiple ne peut pas se trouver entre les carbones en $\alpha$ et en $\beta$ de l'atome d'azote,

caractérisé en ce que l'on hydrolyse un produit de formule (II):

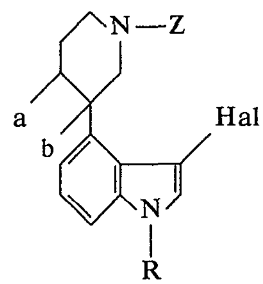

(II)

dans laquelle Hal représente un atome de chlore ou de brome, et R, Z, a et b ont la signification déjà indiquée, pour obtenir un produit de formule générale (I):

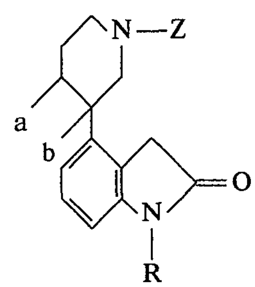

(I)

dans laquelle R, Z, a et b ont la signification déjà indiquée, que l'on isole et salifie si désiré.

2. Variante du procédé selon la revendication 1, caractérisée en ce que l'on hydrolyse un produit de formule (III):

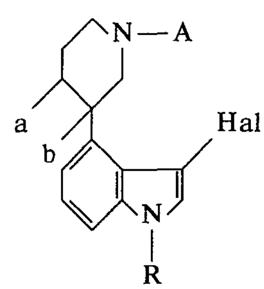

(III)

dans laquelle Hal, R, a et b ont la signification déjà indiquée à la revendication 1 et A représente un atome d'hydrogène, un groupement protecteur éliminable par hydrolyse acide ou un groupement benzyle, pour obtenir un produit de formule ($I_A$):

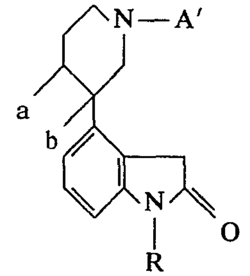

($I_A$)

dans laquelle R, a et b ont la signification déjà indiquée et A' représente un atome d'hydrogène ou un groupement benzyle, correspondant à un produit de formule (I) dans laquelle Z représente un atome d'hydrogène ou un groupement benzyle, composé de formule ($I_A$) que l'on soumet, le cas échéant, à un agent de clivage du groupement benzyle, pour obtenir un produit de formule ($I_B$):

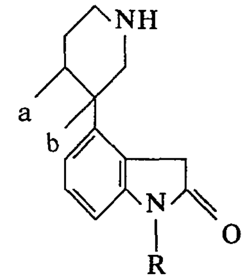

($I_B$)

dans laquelle R, a et b ont la signification déjà indiquée que, le cas échéant, l'on soumet à l'action d'un agent capable d'introduire un radical Z', Z' ayant la même signification que Z, à l'exception d'hydrogène, pour obtenir un produit de formule ($I_C$):

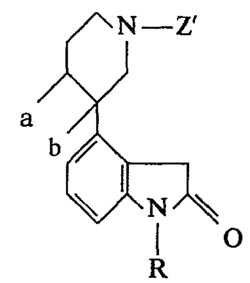

($I_C$)

dans laquelle R, Z', a et b ont la signification déjà indiquée, composé de formule ($I_A$), ($I_B$) ou ($I_C$) que, le cas échéant, l'on salifie.

3. Procédé selon la revendication 1 ou 2, caractérisé en ce que l'hydrolyse est réalisée à l'aide d'un acide minéral en solution aqueuse.

4. Procédé selon la revendication 1, 2 ou 3, caractérisé en ce que l'on utilise au départ un dérivé de formule (II) ou (III), dans laquelle R répresente un atome d'hydogène ou un radical alkyle renfermant de 1 à 8 atomes de carbone.

5. Procédé selon la revendication 1, 2, 3 ou 4, caractérisé en ce que l'on prépare des dérivés de formule (I), dans laquelle Z répresente un atome d'hydrogène ou un radical alkyle renfermant de 1 à 8 atomes de carbone.

6. Procédé selon la revendication 1, 2 ou 3, caractérisé en ce que l'on prépare l'un quelconque des dérivés de formule (I) dont les noms suivent:

- la 1,3-dihydro 4-(1-propyl 1,2,5,6-tétrahydropyridin-3-yl) 2H indol-2-one,
- la 1,3-dihydro 4-(pipéridin-3-yl) 2H indol-2-one,

ainsi que leurs sels d'addition avec les acides minéraux ou organiques.

**Patentansprüche für die Vertragsstaaten: BE, CH, DE, FR, GB, IT, LI, LU, NL, SE**

1. 2-Oxo-indol-Derivate sowie deren Additionssalze mit Mineral- oder organischen Säuren, dadurch gekennzeichnet, daß sie der allgemeinen Formel (I)

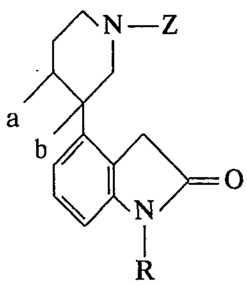

(I)

entsprechen, worin

R ein Wasserstoffatom, einen Alkylrest mit 1 bis 8 Kohlenstoffatomen oder einen Aralkylrest mit 7 bis 12 Kohlenstoffatomen bedeutet,

Z ein Wasserstoffatom, einen Methylrest, einen Alkyl- oder Hydroxyalkylrest mit 2 bis 8 Kohlenstoffatomen, einen Aryloxyalkylrest mit 7 bis 12 Kohlenstoffatomen, einen Aralkylrest mit 7 bis 12 Kohlenstoffatomen, der gegebenenfalls durch ein oder mehrere Halogenatome, durch einen oder mehrere Alkoxyreste mit 1 bis 4 Kohlenstoffatomen, durch einen oder mehrere Alkylreste mit 1 bis 4 Kohlenstoffatomen oder durch einen oder mehrere Reste OH, $CF_3$, $OCF_3$, $NO_2$ oder $NH_2$ substituiert ist, bedeutet oder Z einen Cycloalkylalkylrest mit 4 bis 12 Kohlenstoffatomen, einen Alkenylrest mit 3 bis 8 Kohlenstoffatomen oder einen Alkinylrest mit 3 bis 8 Kohlenstoffatomen bedeutet, und

entweder a und b jeweils ein Wasserstoffatom bedeuten
oder a und b gemeinsam eine Kohlenstoff-Kohlenstoffbindung bilden,
wobei, wenn Z einen Alkenyl- oder Alkinylrest bedeutet, die Mehrfachbindung sich nicht zwischen den Kohlenstoffatomen in $\alpha$- und $\beta$-Stellung zum Stickstoffatom befinden kann.

2. 2-Oxo-indol-Derivate der Formel (I) gemäß Anspruch 1 sowie deren Additionssalze mit Mineral- oder organischen Säuren, dadurch gekennzeichnet, daß R ein Wasserstoffatom oder einen Alkylrest mit 1 bis 8 Kohlenstoffatomen bedeutet.

3. 2-Oxo-indol-Derivate gemäß Anspruch 2 sowie deren Additionssalze mit Mineral- oder organischen Säuren, dadurch gekennzeichnet, daß Z ein Wasserstoffatom oder einen Alkylrest mit 1 bis 8 Kohlenstoffatomen bedeutet.

4. Eines der Derivate der Formel (I) gemäß Anspruch 1 mit den folgenden Bezeichnungen:

1,3-Dihydro-4-(1-propyl-1,2,5,6-tetrahydropyridin-3-yl)-2H-indol-2-on,
1,3-Dihydro-4-(piperidin-3-yl)-2H-indol-2-on

sowie deren Additionssalze mit Mineral- oder organischen Säuren.

5. Verfahren zur Herstellung der 2-Oxo-indol-Derivate der Formel I gemäß Anspruch 1 sowie von deren Additionssalzen mit Mineral- oder organischen Säuren, dadurch gekennzeichnet, daß man ein Produkt der Formel II

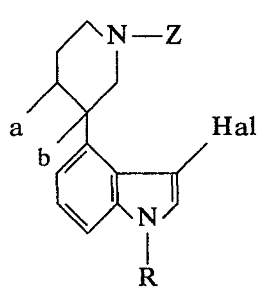

(II)

worin Hal ein Chlor- oder Bromatom bedeutet und R, Z, a und b die angegebene Bedeutung besitzen, hydrolysiert, um ein Produkt der allgemeinen Formel (I)

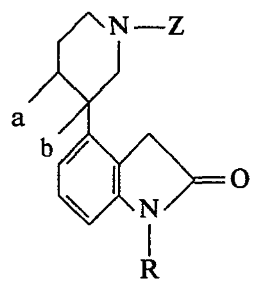

(I)

zu erhalten, worin R, Z, a und b die angegebene Bedeutung besitzen, das man isoliert und gewünschtenfalls in ein Salz überführt.

6. Abänderung des Verfahrens gemäß Anspruch 5, dadurch gekennzeichnet, daß man ein Produkt der Formel (III)

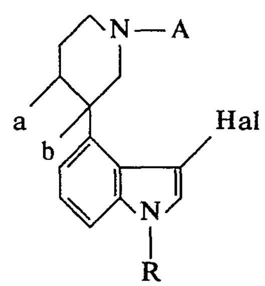

(III)

worin Hal, R, a und b die in Anspruch 5 angegebene Bedeutung besitzen und A ein Wasserstoffatom, eine durch saure Hydrolyse eliminierbare Schutzgruppe oder eine Benzylgruppe bedeutet, hydrolysiert, um ein Produkt der Formel ($I_A$)

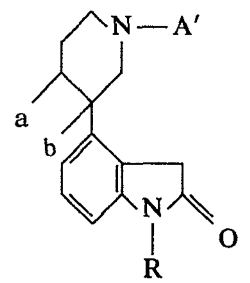

($I_A$)

worin R, a und b die angegebene Bedeutung besitzen und A' ein Wasserstoffatom oder eine Benzylgruppe bedeutet, entsprechend einem Produkt der Formel (I), worin Z ein Wasserstoffatom oder eine Benzylgruppe bedeutet, zu erhalten, welche Verbindung der Formel ($I_A$) man gegebenenfalls einem Mittel für die Abspaltung der Benzylgruppe unterzieht, um ein Produkt der Formel ($I_B$)

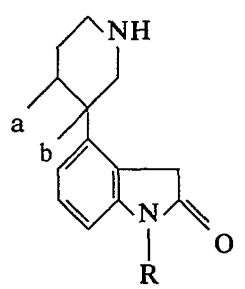

($I_B$)

zu erhalten, worin R, a und b die angegebene Bedeutung besitzen, und gegebenenfalls dieses der Einwirkung eines Mittels unterzieht, das befähigt ist, einen Rest Z' einzuführen, wobei Z' die gleiche Bedeutung wie Z mit Ausnahme von Wasserstoff besitzt, um ein Produkt der Formel ($I_C$)

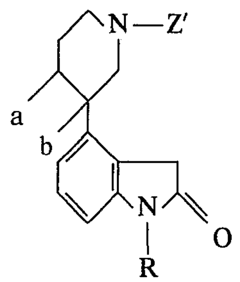

(I_C)

zu erhalten, worin R, Z', a und b die angegebene Bedeutung besitzen, und die Verbindung der Formel ($I_A$), ($I_B$) oder ($I_C$) gegebenenfalls in ein Salz überführt.

7. Verfahren gemäß Anspruch 5 oder 6, dadurch gekennzeichnet, daß die Hydrolyse mit Hilfe einer Mineralsäure in wäßriger Lösung durchgeführt wird.

8. Arzneimittel, dadurch gekennzeichnet, daß sie aus den 2-Oxo-indol-Derivaten der Formel (I) gemäß Anspruch 1 sowie aus deren Additionssalzen mit pharmazeutisch verträglichen Säuren bestehen.

9. Arzneimittel, dadurch gekennzeichnet, daß sie aus den 2-Oxo-indol-Derivaten gemäß einem der Ansprüche 2 oder 3 sowie aus deren Additionssalzen mit pharmazeutisch verträglichen Säuren bestehen.

10. Arzneimittel, dadurch gekennzeichnet, daß sie aus den 2-Oxo-indol-Derivaten gemäß Anspruch 4 sowie aus deren Additionssalzen mit pharmazeutisch verträglichen Säuren bestehen.

11. Pharmazeutische Zusammensetzungen, dadurch gekennzeichnet, daß sie als Wirkstoff zumindest eines der Arzneimittel gemäß einem der Ansprüche 8, 9 oder 10 enthalten.

**Patentansprüche für den Vertragsstaat: AT**

1. Verfahren zur Herstellung von 2-Oxo-indol-Derivaten sowie von deren Additionssalzen mit Mineralsäuren oder organischen Säuren der allgemeinen Formel (I)

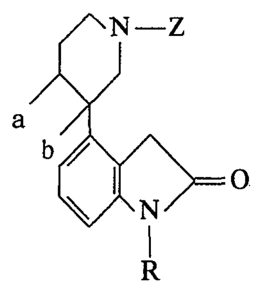

(I)

worin

R ein Wasserstoffatom, einen Alkylrest mit 1 bis 8 Kohlenstoffatomen oder einen Aralkylrest mit 7 bis 12 Kohlenstoffatomen bedeutet,

Z ein Wasserstoffatom, einen Methylrest, einen Alkyl- oder Hydroxyalkylrest mit 2 bis 8 Kohlenstoffatomen, einen Aryloxyalkylrest mit 7 bis 12 Kohlenstoffatomen, einen Aralkylrest mit 7 bis 12 Kohlenstoffatomen, der gegebenenfalls durch ein oder mehrere Halogenatome, durch einen oder mehrere Alkoxyreste mit 1 bis 4 Kohlenstoffatomen, durch einen oder mehrere Alkylreste mit 1 bis 4 Kohlenstoffatomen oder durch einen oder mehrere Reste OH, $CF_3$, $OCF_3$, $NO_2$ oder $NH_2$ substituiert ist, bedeutet oder Z einen Cycloalkylalkylrest mit 4 bis 12 Kohlenstoffatomen, einen Alkenylrest mit 3 bis 8 Kohlenstoffatomen oder einen Alkinylrest mit 3 bis 8 Kohlenstoffatomen bedeutet, und

entweder a und b jeweils ein Wasserstoffatom bedeuten
oder a und b gemeinsam eine Kohlenstoff-Kohlenstoffbindung bilden,
wobei, wenn Z einen Alkenyl- oder Alkinylrest bedeutet, die Mehrfachbindung sich nicht zwischen den Kohlenstoffatomen in $\alpha$- und $\beta$-Stellung zum Stickstoffatom befinden kann,
dadurch gekennzeichnet, daß man ein Produkt der Formel (II)

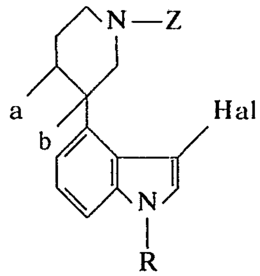

(II)

worin Hal ein Chlor- oder Bromatom bedeutet und R, Z, a und b die angegebene Bedeutung besitzen, hydrolysiert, um ein Produkt der allgemeinen Formel (I)

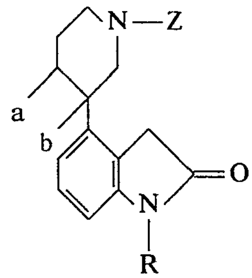

(I)

zu erhalten, worin R, Z, a und b die angegebene Bedeutung besitzen, das man isoliert und gewünschtenfalls in ein Salz überführt.

2. Abänderung des Verfahrens gemäß Anspruch 1, dadurch gekennzeichnet, daß man ein Produkt der Formel (III)

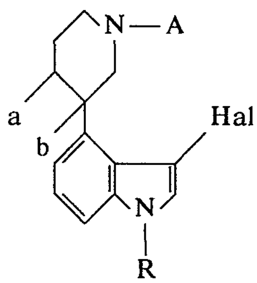

(III)

worin Hal, R, a und b die in Anspruch 1 angegebene Bedeutung besitzen und A ein Wasserstoffatom, eine durch saure Hydrolyse eliminierbare Schutzgruppe oder eine Benzylgruppe bedeutet, hydrolysiert, um ein Produkt der Formel ($I_A$)

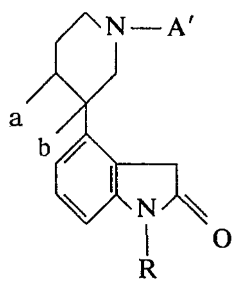

($I_A$)

worin R, a und b die angegebene Bedeutung besitzen und A' ein Wasserstoffatom oder eine Benzylgruppe bedeutet, entsprechend einem Produkt der Formel (I), worin Z ein Wasserstoffatom oder eine Benzylgruppe bedeutet, zu erhalten, welche Verbindung der Formel ($I_A$) man gegebenenfalls der Einwirkung eines Mittels zur Abspaltung der Benzylgruppe unterzieht, um ein Produkt der Formel ($I_B$)

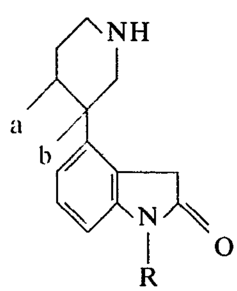

($I_B$)

zu erhalten, worin R, a und b die angegebene Bedeutung besitzen, welches man gegebenenfalls der Einwirkung eines Mittels zur Einführung eines Restes Z' unterzieht, wobei Z' die gleiche Bedeutung wie Z mit Ausnahme von Wasserstoff besitzt, um ein Produkt der Formel ($I_C$)

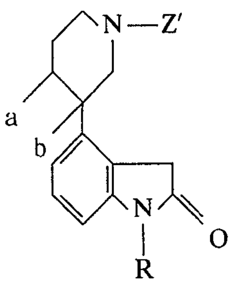

($I_{C'}$)

zu erhalten, worin R, Z', a und b die angegebene Bedeutung besitzen, und die Verbindung der Formel ($I_A$), ($I_B$) oder ($I_C$) gegebenenfalls in ein Salz überführt.

3. Verfahren gemäß Anspruch 1 oder 2, dadurch gekennzeichnet, daß die Hydrolyse mit Hilfe einer Mineralsäure in wäßriger Lösung durchgeführt wird.

4. Verfahren gemäß Anspruch 1, 2 oder 3, dadurch gekennzeichnet, daß man von einem Derivat der Formel (II) oder (III) ausgeht, worin R ein Wasserstoffatom oder einen Alkylrest mit 1 bis 8 Kohlenstoffatomen bedeutet.

5. Verfahren gemäß Anspruch 1, 2, 3 oder 4, dadurch gekennzeichnet, daß man Derivate der Formel (I) herstellt, worin Z ein Wasserstoffatom oder einen Alkylrest mit 1 bis 8 Kohlenstoffatomen bedeutet.

6. Verfahren gemäß Anspruch 1, 2 oder 3, dadurch gekennzeichnet, daß man eines der Derivate der Formel (I) mit den folgenden Bezeichnungen herstellt:

1,3-Dihydro-4-(1-propyl-1,2,5,6-tetrahydropyridin-3-yl)-2H-indol-2-on,
1,3-Dihydro-4-(piperidin-3-yl)-2H-indol-2-on

sowie deren Additionssalze mit Mineral- oder organischen Säuren.

**Claims for the contracting states: BE, CH, DE, FR, GB, IT, LI, LU, NL, SE**

1. The derivatives of 2-oxo-indole, as well as their salts of addition with mineral or organic acids, characterized in that they answer to the general formula (I):

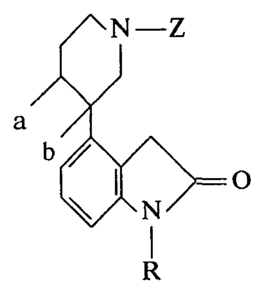

(I)

in which

- R represents a hydrogen atom, an alkyl radical containing from 1 to 8 carbon atoms, or an aralkyl radical containing from 7 to 12 carbon atoms,
- Z represents a hydrogen atom, a methyl radical, an alkyl or hydroxyalkyl radical containing from 2 to 8 carbon atoms, an aryloxyalkyl radical containing from 7 to 12 carbon atoms, an aralkyl radical containing from 7 to 12 carbon atoms possibly substituted by one or more halogen atoms, by one or more alkoxy radicals containing from 1 to 4 carbon atoms, by one or more alkyl radicals containing from 1 to 4 carbon atoms, or by one or more radicals OH, $CF_3$, $OCF_3$, $NO_2$ or $NH_2$, or Z represents a cycloalkyl-alkyl radical containing from 4 to 12 carbon atoms, an alkenyl radical containing from 3 to 8 carbon atoms or an alkynyl radical containing from 3 to 8 carbon atoms and,
- either a and b each represent a hydrogen atom,
- or a and b together form a carbon-carbon bond, it being understood that when Z represents an alkenyl or alkynyl radical, the multiple bond can not be situated between the carbons at $\alpha$ and $\beta$ of the nitrogen atom.

2. The derivatives of 2-oxo-indole answering to the formula (I) as defined in claim 1, as well as their

salts of addition with mineral or organic acids, characterized in that R represents a hydrogen atom or an alkyl radical containing from 1 to 8 carbon atoms.

3. Derivates of 2-oxo-indole according to claim 2, as well as their salts of addition with mineral or organic acids, characterized in that Z represents a hydrogen atom or an alkyl radical containing from 1 to 8 carbon atoms.

4. Any one of the derivatives with the formula (I), as defined in claim 1, the names of which follow:

— 1,3-dihydro-4-(1-propyl-1,2,5,6-tetrahydropyridin-3-yl) 2H indol-2-one,
— 1,3-dihydro-4-(piperidin-3-yl) 2H indol-2-one,

as well as their salts of addition with mineral or organic acids.

5. Preparation process of derivatives of 2-oxo-indole with the formula I as defined in claim 1, as well as their salts of addition with mineral or organic acids, characterized in that a product with the formula II:

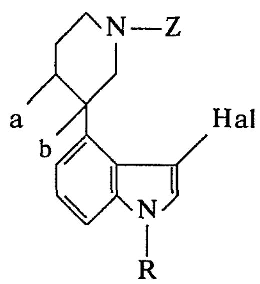

(II)

in which Hal represents a chlorine or bromine atom, and R, Z, a and b have the significance already indicated, is hydrolyzed so as to obtain a product with the general formula (I):

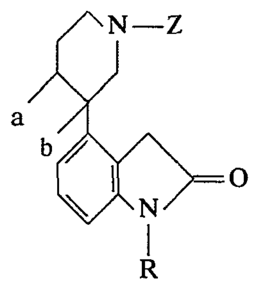

(I)

in which R, Z, a and b have the significance already indicated, which is isolated and salified if desired.

6. Variant of the process according to claim 5, characterized in that a product with the formula (III):

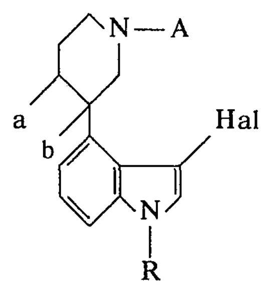

(III)

in which Hal, R, a and b have the significance already indicated in claim 5 and A represents a hydrogen atom, a protector group which can be eliminated by acid hydrolysis or a benzyl group, is hydrolyzed so as to obtain a product with the formula ($I_A$):

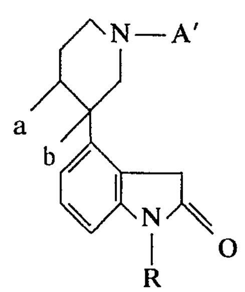

($I_A$)

in which R, a and b have the significance already indicated and A′ represents a hydrogen atom or a benzyl group, corresponding to a product with the formula (I) in which Z represents a hydrogen atom or a benzyl group, which compound with the formula ($I_A$) is submitted, if the case arises, to a cleavage agent of the benzyl group, so as to obtain a product with the formula ($I_B$):

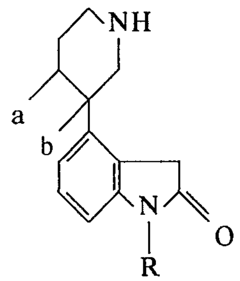

($I_B$)

in which R, a and b have the significance already indicated, and which, if the case arises, is submitted to the action of an agent capable of introducing a radical Z′, Z′ having the same significance as Z, with the exception of hydrogen, so as to obtain a product with the formula ($I_C$):

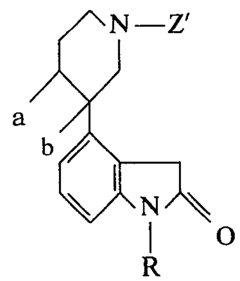

($I_C$)

in which R, Z′, a and b have the significance already indicated, which compound with the formula ($I_A$), ($I_B$) or ($I_C$), if the case arises, is salified.

7. Process according to claim 5 or 6, characterized in that hydrolysis is carried out by a mineral acid in an aqueous solution.

8. Medicaments, characterized in that they are composed of derivatives of 2-oxo-indole as defined by formula (I) of claim 1, as well as by their salts of addition with pharmaceutically acceptable acids.

9. Medicaments, characterised in that they are composed of derivatives of 2-oxo-indole, as defined in one of the claims 2 or 3, as well as by their salts of addition with pharmaceutically acceptable acids.

10. Medicaments, characterized in that they are composed of derivatives of 2-oxo-indole, as defined in claim 4, as well as by their salts of addition with pharmaceutically acceptable acids.

11. Pharmaceutical compositions, characterized in that they contain, as active principle, at least one of the medicaments, as defined in one of the claims 8, 9 or 10.

**Claims for the contracting state: AT**

1. Preparation process of derivatives of 2-oxo-indole, as well as their salts of addition with mineral or organic acids, answering to the general formula (I):

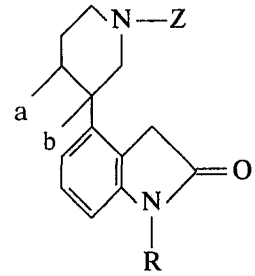

(I)

in which

– R represents a hydrogen atom, an alkyl radical containing from 1 to 8 carbon atoms, or an aralkyl radical containing from 7 to 12 carbon atoms,
– Z represents a hydrogen atom, a methyl radical, an alkyl or hydroxyalkyl radical containing from 2 to 8 carbon atoms, an aryloxyalkyl radical containing from 7 to 12 carbon atoms, an aralkyl radical containing from 7 to 12 carbon atoms possibly substituted by one or more halogen atoms, by one

or more alkoxy radicals containing from 1 to 4 carbon atoms, by one or more alkyl radicals containing from 1 to 4 carbon atoms, or by one or more radicals OH, $CF_3$, $OCF_3$, $NO_2$ or $NH_2$, or Z represents a cycloalkyl-alkyl radical containing from 4 to 12 carbon atoms, an alkenyl radical containing from 3 to 8 carbon atoms or an alkynyl radical containing from 3 to 8 carbon atoms and,

- either a and b each represent a hydrogen atom,
- or a and b together form a carbon-carbon bond, it being understood that when Z represents an alkenyl or alkynyl radical, the multiple bond can not be situated between the carbons at $\alpha$ and $\beta$ of the nitrogen atom,

characterized in that a product with the formula (II):

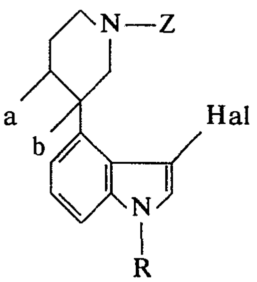

(II)

in which Hal represents a chlorine or bromine atom, and R, Z, a and b have the significance already indicated, is hydrolyzed so as to obtain a product with the general formula (I):

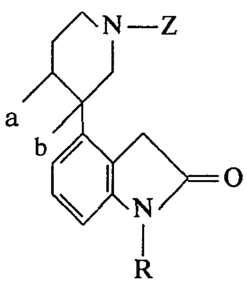

(I)

in which R, Z, a and b have the significance already indicated, which is isolated and salified if desired.

2. Variant of the process according to claim 1, characterized in that a product with the formula (III):

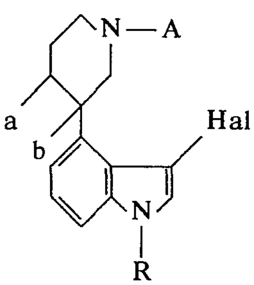

(III)

in which Hal, R, a and b have the significance already indicated in claim 1 and A represents a hydrogen atom, a protector group which can be eliminated by acid hydrolysis or a benzyl group, is hydrolyzed so as to obtain a product with the formula ($I_A$):

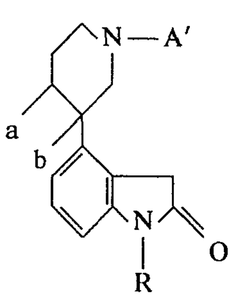

($I_A$)

in which R, a and b have the significance already indicated and A′ represents a hydrogen atom or a benzyl group, corresponding to a product with the formula (I) in which Z represents a hydrogen atom or a benzyl group, which compound with the formula ($I_A$) is submitted, if the case arises, to a cleavage agent of the benzyl group, so as to obtain a product with the formula ($I_B$):

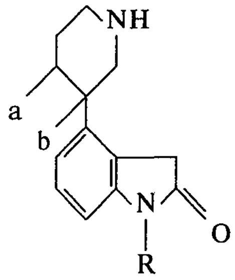

($I_B$)

in which R, a and b have the significance already indicated, and that if the case arises, is submitted to the action of an agent capable of introducing a radical Z′, Z′ having the same significance as Z, with the exception of hydrogen, so as to obtain a product with the formula ($I_C$):

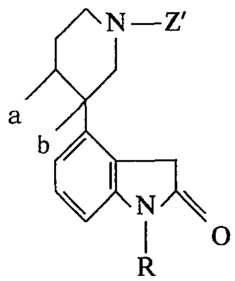

($I_C$)

in which R, Z′, a and b have the significance already indicated, which compound with the formula ($I_A$), ($I_B$) or ($I_C$), if the case arises, is salified.

3. Process according to claim 1 or 2, characterized in that hydrolysis is carried out by a mineral acid in an aqueous solution.

4. Process according to claims 1, 2 or 3, characterized in that at the start a derivative with the formula (II) or (III) is used, in which R represents a hydrogen atom or an alkyl radical containing from 1 to 8 carbon atoms.

5. Process according to claims 1, 2, 3 or 4, characterized in that derivatives with the formula (I) are prepared, in which Z represents a hydrogen atom or an alkyl radical containing from 1 to 8 carbon atoms.

6. Process according to claims 1, 2 or 3, characterised in that any one of the derivatives with the formula (I), are prepared, the names of which follow:

– 1,3-dihydro-4-(1-propyl-1,2,5,6-tetrahydropyridin-3-yl) 2H indol-2-one,
– 1,3-dihydro-4-(piperidin-3-yl) 2H indol-2-one,

as well as their salts of addition with mineral or organic acids.